(19) Europäisches Patentamt
European Patent Office
Office européen des brevets

(11) EP 4 205 746 A1

(12) **EUROPEAN PATENT APPLICATION**
published in accordance with Art. 153(4) EPC

(43) Date of publication:
**05.07.2023 Bulletin 2023/27**

(21) Application number: **21860587.1**

(22) Date of filing: **01.09.2021**

(51) International Patent Classification (IPC):
***A61K 31/519*** (2006.01) ***A61K 31/5365*** (2006.01)
***A61P 35/00*** (2006.01)

(52) Cooperative Patent Classification (CPC):
**A61K 31/519; A61K 31/5365; A61P 35/00**

(86) International application number:
**PCT/CN2021/116076**

(87) International publication number:
**WO 2022/042755 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.08.2020 CN 202010889864**

(71) Applicant: **East China University of Science and Technology**
**Shanghai 200237 (CN)**

(72) Inventors:
- **LI, Honglin**
  **Shanghai 200237 (CN)**
- **ZHAO, Zhenjiang**
  **Shanghai 200237 (CN)**
- **DIAO, Yanyan**
  **Shanghai 200237 (CN)**
- **CHEN, Zhuo**
  **Shanghai 200237 (CN)**

(74) Representative: **Mewburn Ellis LLP**
**Aurora Building**
**Counterslip**
**Bristol BS1 6BX (GB)**

# (54) COMPOUND FOR INHIBITING MUTANT EGFR AND USE THEREOF

(57) Provided is the use of a series of pteridinones and/or a pharmaceutically acceptable salt and a prodrug thereof as a non-canonical EGFR mutant inhibitor. Specifically, the present invention relates to the use of a series of compounds as represented by formula I and a pharmaceutical composition containing the series of compounds as represented by formula I in the preparation of a drug for treating a disease containing EGFR 20insX mutation, EGFR G719X mutation and ERBB2 mutation.

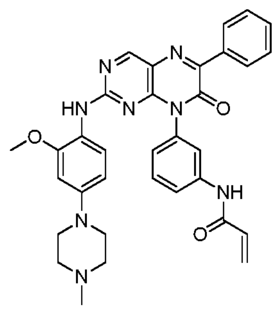

I

EP 4 205 746 A1

## Description

## TECHNICAL FIELD

The present invention relates to the field of pharmaceutical chemistry. In particular, the present invention relates to the use of pteridinone derivatives as inhibitors of the mutant epidermal growth factor receptor tyrosine kinase (EGFR), in particular non-classical mutant EGFR.

## Background

As the first, second and third generation of EGFR inhibitors marketed, it brings good news to non-small cell lung cancer patients with EGFR typical sensitive mutations and T790M drug-resistant mutation. However, not all patients with EGFR mutation can be benefited. The NSCLC of EGFR 20 exon insertion mutation (EGFR 20ins) exhibits poor targeted therapy effects on most EGFR inhibitors, and these mutations are generally classified as non-classical mutations. These non-classical mutations mainly include the insertion mutation and point mutation of the exon 18-21, and the point mutation and the insertion mutation of ErbB 2.

Therefore, the research and development of drugs targeting EGFR with non-classical mutations has great clinical significance and application prospects.

## Summary of the invention

An object of the present invention is to provide an inhibitor on EGFR with non-classical mutations and a pharmaceutical composition comprising the inhibitor.

In a first aspect, the present invention provides the use of a compound of formula I or a pharmaceutically acceptable salt thereof in the preparation of a drug for inhibiting mutant EGFR or a drug for treating or preventing mutant EGFR-mediated diseases:

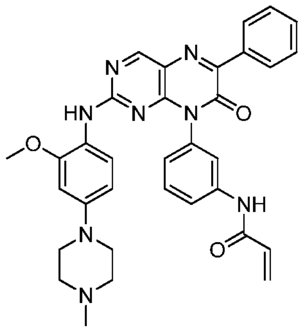

I.

In a specific embodiment, the mutant EGFR comprises at least one of the following mutations: point mutation and insertion mutation of EGFR 18-21 exon, and point mutation and insertion mutation of ErbB 2.

In a specific embodiment, the mutant EGFR comprises:

18 exon G719X, E709X, K716A, K728A point mutations and deletion mutation at codon 709;

19 exon insertion mutation I744-K745InsKIPVAI, K745-E746insIPVAIK, K745-E746insVPVAIK, K745-E746insTPVAIK and point mutation D761Y;

20 exon insertion mutation and point mutation include: A763-Y764insFQEA, A763-Y764insFHEA, V769-D770insASV, V769-D770insDNP , D770-N771insNPG, D770-N771insNPH, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV and the like, and 20 exon point mutation S768I;

21 exon point mutation L861Q;

ERBB2 point mutations V777L, D769Y, R896C, P 1170A and insertion mutations V777-G778insCG, P780-Y781insGSP, and the like.

In a specific embodiment, the mutant EGFR comprises at least one of the following mutations: G719X (X represents A, S, C, D), D761Y, A763-Y764insFQEA, A763-Y764insFHEA, V769-D770insASV, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV, L861Q, V777-G778insCG, V777L, D769Y and the like.

In a specific embodiment, the mutant EGFR comprises at least one of the following mutations: A763_Y764insFHEA, A763-Y764insFQEA, d747-749/A750P D761Y, D770-N771insNPG, D770-N771insNPG/T790M, D770GY, G719C, G719D, G719S, and L861Q, and V777-G778insCG, V777L, D769Y of ERBB, and the like.

In a specific embodiment, the disease is a cancer.

In a specific embodiment, the cancer is non-small cell lung cancer, small cell lung cancer, lung adenocarcinoma, lung squamous cell carcinoma, breast cancer, pancreatic cancer, prostate cancer, ovarian cancer, glioblastoma, head and neck squamous cell carcinoma, cervical cancer, esophageal cancer, liver cancer, kidney cancer, colon cancer, skin cancer, leukemia, lymphoma, gastric cancer or multiple myeloma.

In a second aspect, the present invention provides a compound of formula I or a pharmaceutically acceptable salt thereof, for inhibiting mutant EGFR or treating or preventing mutant EGFR-mediated diseases,

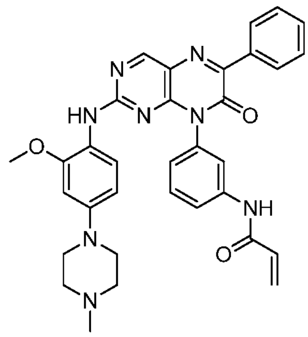

I.

In a preferred embodiment, the mutant EGFR comprises at least one of following mutations: point mutation and insertion mutation of EGFR 18-21 exon, and point mutation and insertion mutation of ErbB 2.

In a preferred embodiment, the mutant EGFR comprises:

18 exon G719X, E709X, K716A, K728A point mutations and deletion mutation at codon 709;

19 exon insertion mutations I744-K745InsKIPVAI, K745-E746insIPVAIK, K745-E746insVPVAIK, K745-E746insTPVAIK and point mutation D761Y;

20 exon insertion mutation and point mutation include: A763-Y764insFQEA, A763-Y764insFHEA, V769-D770insASV, V769-D770insDNP , D770-N771insNPG, D770-N771insNPH, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV and the like, and 20 exon point mutation S768I;

21 exon point mutation L861Q;

ERBB2 point mutations V777L, D769Y, R896C, P 1170A and insertion mutations V777-G778insCG, P780-Y781insGSP, and the like.

In a preferred embodiment, the mutant EGFR comprises at least one of the following mutations: G719X (X represents A, S, C, D), D761Y, A763-Y764insFQEA, A763-Y764insFHEA, V769-D770insASV, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV, L861Q, V777-G778insCG, V777L, D769Y and the like.

In a preferred embodiment, the mutant EGFR comprises at least one of the following mutations: A763_Y764insFHEA, A763-Y764insFQEA, d747-749/A750P D761Y, D770-N771insNPG, D770-N771insNPG/T790M, D770GY, G719C, G719D, G719S, and L861Q, and V777-G778insCG, V777L, D769Y of ERBB, and the like.

In a preferred embodiment, the disease is a cancer.

In a preferred embodiment, the cancer is non-small cell lung cancer, small cell lung cancer, lung adenocarcinoma, lung squamous cell carcinoma, breast cancer, pancreatic cancer, prostate cancer, ovarian cancer, glioblastoma, head and neck squamous cell carcinoma, cervical cancer, esophageal cancer, liver cancer, kidney cancer, colon cancer, skin cancer, leukemia, lymphoma, gastric cancer or multiple myeloma.

In a third aspect, the present invention provides a method for inhibiting mutant EGFR or treating or preventing mutant EGFR-mediated diseases, comprising a step of administering a compound of formula I or a pharmaceutically acceptable salt thereof to a subject in need thereof,

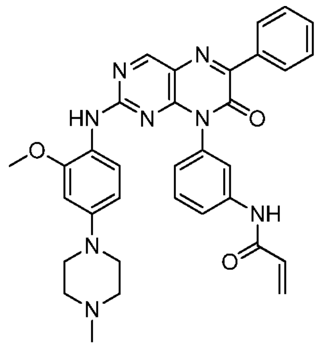

I.

In a preferred embodiment, the mutant EGFR comprises at least one of following mutations: point mutation and insertion mutation of EGFR 18-21 exon, and point mutation and insertion mutation of ErbB 2.

In a preferred embodiment, the mutant EGFR comprises:

18 exon G719X, E709X, K716A, K728A point mutations and deletion mutation at codon 709;
19 exon insertion mutations I744-K745InsKIPVAI, K745-E746insIPVAIK, K745-E746insVPVAIK, K745-E746insTPVAIK and point mutation D761Y;
20 exon insertion mutation and point mutation include: A763-Y764insFQEA, A763-Y764insFHEA, V769-D770insASV, V769-D770insDNP , D770-N771insNPG, D770-N771insNPH, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV and the like, and 20 exon point mutation S768I;
21 exon point mutation L861Q;
Point mutations V 777 L, D 769 Y, R 896 C, P 1170 A and insertion mutations V 777-G 778 INS1, P780-Y781 INSGSP, etc. of ErbB 2.

ERBB2 point mutations V777L, D769Y, R896C, P 1170A and insertion mutations V777-G778insCG, P780-Y781insGSP, and the like.

In a preferred embodiment, the mutant EGFR comprises at least one of the following mutations: G719X (X represents A, S, C, D), D761Y, A763-Y764insFQEA, A763-Y764insFHEA, V769-D770insASV, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV, L861Q, V777-G778insCG, V777L, D769Y and the like.

In a preferred embodiment, the mutant EGFR comprises at least one of the following mutations: A763_Y764insFHEA, A763-Y764insFQEA, d747-749/A750P D761Y, D770-N771insNPG, D770-N771insNPG/T790M, D770GY, G719C, G719D, G719S, and L861Q, and V777-G778insCG, V777L, D769Y of ERBB, and the like.

In a preferred embodiment, the disease is a cancer.

In a preferred embodiment, the cancer is non-small cell lung cancer, small cell lung cancer, lung adenocarcinoma, lung squamous cell carcinoma, breast cancer, pancreatic cancer, prostate cancer, ovarian cancer, glioblastoma, head and neck squamous cell carcinoma, cervical cancer, esophageal cancer, liver cancer, kidney cancer, colon cancer, skin cancer, leukemia, lymphoma, gastric cancer or multiple myeloma.

It should be understood that, in the scope of the present disclosure, the above-mentioned technical features of the present disclosure and the technical features specifically described in the following (for example, Examples) may be combined with each other, thereby constituting a new or preferred technical solution, which is not listed herein due to the limitation on contents.

## Brief Description of Drawings

Figures 1a-f show GI50 data from the proliferation inhibition test for stable cell lines containing different EGFR mutations by compound I, wherein compound 1 is shown as -R 201, and compound TAK788 is shown as R-203.

## Modes for carrying out the invention

Upon extensive and in-depth research, the inventors unexpectedly found pteridinone derivatives capable of targeting non-classical mutant EGFR, and thus can be used as a new generation of EGFR inhibitors, based on which

the present is completed.

**EGFR non-classical mutation**

EGFR inhibitors bring good news to non-small cell lung cancer patients with EGFR typical sensitive mutations and T790M drug-resistant mutation. However, not all patients with EGFR mutation can be benefited from the marketed EGFR inhibitors. It has been found that the NSCLC with EGFR 20 exon insertion mutation (EGFR 20ins) exhibits poor targeted therapy effects on most EGFR inhibitors. These mutations are generally classified as non-classical mutations. In the present invention, these non-classical mutations mainly include the insertion mutation and point mutation of the exon 18-21, and the point mutation and the insertion mutation of ErbB 2.

The non-classical mutations of exon 18 are mainly G719X, E709X, K7716A, K728A point mutations, and a deletion mutation at codon 709. G719X was mutated to G719X in EGFR (X represents A alanine, S serine, C cysteine, etc.), which is a point mutation, and G719X mutation accounts for about 3.10% of total EGFR mutation [Cancer Sci. 2016, 107(9),: 1179-1186].

The carcinogenicity of G719S mutation is weaker than that of sensitive mutation, and it is found through *in vitro* experiments that Gefitinib can inhibit the phosphorylation of G719S in a dosage-increasing manner. Higher concentration of Gefitinib is required to inhibit G719S mutation as compared with the sensitive mutation L858R. E709X point mutation is another non-classical mutation of exon 18, and E709X mutation accounts for about 0.30% of the total EGFR mutations. In *in vitro* experiments, patients with E709X point mutation exhibit high sensitivity to Afatinib, compared with the first generation or third generation of TKIs.

DelE709-T710insD is the most common deletion mutation at codon 709, accounting for about 0.30% of the total EGFR mutations.

Non-classical mutations of exon 19 are insertion mutation and point mutation. The insertion mutation of exon 19 accounts for about 0.60% of the EGFR mutations, including: I744-K745insKIPVAI, K745-E746insIPVAIK, K745-E746insVPVAIK, K745-E746insTPVAIK. *In vitro* tests showed that insertion mutations of exon 19 are sensitive to Afatinib.

The main point mutation of exon 19 is D761Y, and it is believed that its appearance may be related to EGFR-TKI drug resistance, however, the drug resistance level is weaker than that of point mutation T790M of exon 20. And at present, there is no clinical trial data.

Amino acid sites translated by exon 20 is 762-823. Glu762, starting at N-terminal, is an important catalytic site. It comprises a C-helix consisting of amino acids E762-M766 and a ring consisting of amino acids A767-V774. The non-classical mutations of exon 20 are mainly insertion mutation and point mutation, inchluding: A763-Y764insFQEA, V769-D770insASV, V769-D770insDNP , D770-N771insNPG, D770-N771insNPH, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV, and the like. The above mutations account for about 5.80% of the total EGFR mutations. In addition to A763-Y764insFQEA, the insertion mutation of exon 20 of EGFR is insensitive to the 1$^{st}$, 2$^{nd}$ generation of TKIs targeted therapy. Among numerous mutation forms, 763_764insFQEA is the most special. 763_764insFQEA is a sensitive mutation currently found in ex20ins mutations.

So far, more than 60 unique forms of EGFR ex20ins have been found, and the most common form of EGFR ex20ins in the study is D770_N771>ASVDN, N771_P772>SVDNP and N771_H773upNPH, accounting for about 50% of the ex20ins mutations.

In recent *in vitro* experiments, Poziotinib was found to effectively inhibit the growth of Ba/F3 cell lines with an insertion mutation of EGFR exon 20. Poziotinib was stronger than Osimertinib and Afatinib. However, side effects were evident in the form of rash and diarrhea, and clinical trial data are absent.

The point mutation of exon 20 is mainly: S768I, which accounts for about 1.0% of the total EGFR mutations. In *in vitro* experiments, S768I mutation was more sensitive to Afatinib as compared with Osimertinib.

EGFR ex20ins mutations are not as sensitive to the first and second generation EGFR-TKIs as classical mutations, but are the most common non-sensitive mutations among EGFR mutations. Therefore, the treatment of people with ex20ins mutations is currently a major challenge for clinicians in clinical management.

The non-classical mutation of exon 21 is mainly characterized by the point mutation L861Q, which accounts for about 0.90% of the total EGFR mutations. L861Q mutation is caused by the replacment of T by A at site 2828 of exon 21 and has similar oncogenic activity to that of L858R mutation. It was found through *in vitro studies* the L861Q mutation is sensitive to Osimertinib, but clinical trial data are absent.

ERBB2 point mutations V777L, D769Y, R896C, P 1170A and insertional mutations V777-G778insCG and P780-Y781insGSP.

**Compounds and Pharmaceutical Compositions of the Invention**

The compound of the present invention is a compound of formula I or a pharmaceutically acceptable salt thereof,

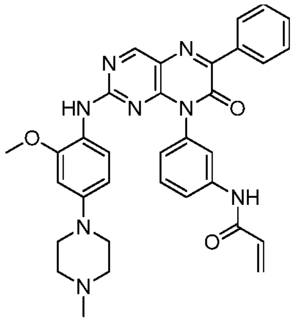

I.

The preparation method of the compound of the present invention can be the method described in CN108721298A.

The compound of the present invention can inhibit mutant EGFR, in particular point mutation and insertion mutation of EGFR exon 18-21, as well as point mutation and insertion mutation of ErbB2. Therefore, based on the compounds of the present invention, the present invention also provides a pharmaceutical composition comprising a therapeutically effective amount of a compound of the invention or a pharmaceutically acceptable salt thereof, and a pharmaceutically acceptable carrier or excipient.

Examples of pharmaceutically acceptable salts of the compound of the present invention include, but are not limited to, inorganic and organic acid salts such as hydrochloride, hydrobromate, sulfate, citrate, lactate, tartrate, maleate, fumarate, mandelate, and oxalate; and inorganic and organic base salts formed with bases such as sodium hydroxyl, tris (hydroxymethyl) aminomethane (Tris, amine butantriol) and N-methylglucamine.

The pharmaceutical compositions of the present invention can be formulated into formulations suitable for various administration routes, including but not limited to the form of administration for parenteral, subcutaneous, intravenous, intramuscular, intraperitoneal, transdermal, oral, intrathecal, intracranial, nasal or external pathway for the treatment of tumors and other diseases. The dosage is an amount effective to improve or eliminate one or more conditions. For the treatment of a particular disease, the effective amount is an amount sufficient to improve or mitigate symptoms associated with a disease in certain ways. Such dosage may be administered as a single dose, or may be administered in accordance with an effective treatment regimen. The amount of administration may be able to cure a disease, but is generally intended to improve the symptoms of the disease. Repeated administrations are generally needed to achieve the desired improvement on symptoms. The dosage of a drug will be determined according to the age, health and weight of a patient, the type of parallel therapy, the frequency of therapy, and desired therapeutic benefits.

The pharmaceutical formulations of the present invention can be administered to any mammal so long as they can obtain the therapeutic effects of the compound of the invention. Most important in these mammals is human.

Based on the teachings of the present invention, a skilled person in the art will appreciate that the compound of the present invention may be used to treat a cancer. In a specific embodiment, the cancer is non-small cell lung cancer, small cell lung cancer, lung adenocarcinoma, lung squamous cell carcinoma, breast cancer, pancreatic cancer, prostate cancer, ovarian cancer, glioblastoma, head and neck squamous cell carcinoma, cervical cancer, esophageal cancer, liver cancer, kidney cancer, colon cancer, skin cancer, leukemia, lymphoma, gastric cancer, or multiple myeloma.

The pharmaceutical formulations of the present invention can be manufactured in a known manner. For example, the formulation can be prepared by traditional mixing, pelletizing, ingot making, dissolution, or freeze drying processes. When an oral formulation is manufactured, the mixture may be selectively ground in combination with solid excipients and the active compound. If necessary, an appropriate amount of auxiliary agent can be added, and the particle mixture is processed to obtain a tablet or lozenge core.

Suitable excipients are fillers, for example sugars, such as lactose or sucrose, mannitol or sorbitol; cellulosic or calcium phosphates, such as tricalcium phosphate or calcium hydrophosphate; and binders, such as starch pastes, including corn starch, wheat starch, rice starch, potato starch, gelatin, astragalus, methylcellulose, hydroxypropyl methylcellulose, sodium carboxymethylcellulose, or polyvinylpyrrolidone. If necessary, a disintegrant, such as the starch mentioned above, and carboxymethyl starch, crosslinked polyvinylpyrrolidone, agar, or alginic acid or a salt thereof, such as sodium alginate, may be added. Auxiliary agents, in particular flow modulators and lubricants, for example, silica, talc, stearate, such as calcium magnesium stearate, stearic acid, or polyethylene glycol. If necessary, the lozenge core may be appropriately coated to resists gastric juice. For this purpose, a concentrated saccharide solutions can be applied. This solution may contain arabic gum, talc, polyvinylpyrrolidone, polyethylene glycol and/or titanium dioxide, a

paint solution, and a suitable organic solvent or solvent mixture. To prepare a gastric-resistant coating, an appropriate cellulose solution, such as cellulose acetate phthalic acid or hydroxypropyl methyl cellulose phthalic acid, may be used. The dye or pigment may be added to the coating of a tablet or lozenge core, for example, a combination for identifying or for characterizing the dosage of an active ingredient.

**Advantages of the present invention:**

1. The compound provided in the present invention is a brand-new compound capable of inhibiting mutant EGFRs, especially non-classical mutant EGFRs;
2. The compound provided in the present invention has excellent inhibitory activities against non-classical mutant EGFRs;
3. The compound provided in the present invention lays a foundation for the development of drugs capable of inhibiting EGFR mutations, especially non-classical mutant EGFRs, and has great industrialization and commercialization prospects and market value, and has significant economic benefits.

The technical solutions of the present invention are further described below in connection with specific embodiments, however following embodiments do not constitute a limitation on the present invention and all the various methods of application employed according to the principles and technical means of the present invention fall within the scope of the present invention. Experimental methods for which specific conditions are not indicated in the following embodiments are usually performed according to conventional conditions, or according to the conditions recommended by the manufacturer. Percentages and parts are calculated by weight unless otherwise noted.

**Example 1**

Compound 1 Kinase Spectrum Evaluation (Commissioned Test: Reaction Biology Corporation, One Great Valley Parkway, SUTE 2 Malvern, PA 19355, USA.)

Experimental Method:
The substrate for the kinase reaction was placed in a freshly prepared reaction buffer and the co-factors required for the kinase reaction were added, and the buffer was prepared as: 20 mM HEPES (pH 7.5), 10 mM $MgCl_2$, 1 mM EGTA, 0.02% Brij35, 0.02 mg/mL BSA, 0.1 mM $Na_3VO_4$, 2 mM DTT, 1% DMSO; afterwards, the kinase to be tested was added and gently shaken. The compound to be tested was dissolved in DMSO to the desired concentration and added to the above buffer containing the kinase/substrate using an autosampler Echo550. $^{33}P$-labeled ATP ($^{33}P$-ATP at a final concentration of 0.01 μCi/μL) was added to the reaction system to initiate the kinase reaction at room temperature for 2 h. The reaction system was spotted on a P81 ion exchange paper (Whatman #3698-915), thoroughly washed and filtered with 0.75% phosphoric acid, and finally the radioactive phosphorylated substrate remaining on the filter paper was measured. Kinase activity data were expressed as a percentage of the remaining kinase activity in the test sample, 10 concentration gradients were measured, a curve was fitted and $IC_{50}$ values were calculated using Prism4 (GraphPad software).

$IC_{50}$ Values of Compound 1 on EGFR Mutant Kinase Spectrum (nM):

| **Kinase** | Compound I | Osimertinib | Afatinib | Poziotinib | Staurosporine |
|---|---|---|---|---|---|
| **EGFR** | 0.08 | 0.37 | 0.06 | | 134.00 |
| **EGFR (A763_Y764insFHEA)** | 0.40 | 0.25 | 0.19 | | 126.00 |
| **EGFR (A763_Y764insFQEA)** | 0.29 | 0.30 | 0.26 | | 120.00 |
| **EGFR (d746-750)** | 0.84 | 0.71 | 0.61 | | 30.30 |
| **EGFR (d747-749/A750P)** | 0.29 | 0.28 | 0.18 | | 23.50 |
| **EGFR (D761Y)** | 0.12 | 0.39 | 0.09 | | 72.30 |
| **EGFR (D770_N771insNPG)** | 0.23 | 0.49 | 0.29 | | 56.20 |

(continued)

| Kinase | Compound I | Osimertinib | Afatinib | Poziotinib | Staurosporine |
|---|---|---|---|---|---|
| **EGFR (D770_N771insNPG/T790M)** | 0.49 | 0.17 | 0.81 | | 2.11 |
| **EGFR (D770GY)** | 0.14 | 0.54 | 0.09 | | 149.00 |
| **EGFR (G719C)** | 0.51 | 1.68 | 0.49 | | 345.00 |
| **EGFR (G719D)** | 0.28 | 4.96 | 0.13 | | 334.00 |
| **EGFR (G719S)** | 0.36 | 4.08 | 0.29 | | 1250.00 |
| **EGFR (L858R)** | 0.17 | 0.22 | 0.11 | | 42.20 |
| **EGFR (L858R, T790M)** | 0.34 | 0.14 | 1.15 | | 2.14 |
| **EGFR (L861Q)** | 0.15 | 0.33 | 0.12 | | 190.00 |
| **ERBB2 (D769Y)** | 0.35 | | 0.31 | 0.21 | 19.20 |
| **ERBB2 (V777_G778insCG)** | 0.22 | | 0.22 | 0.09 | 94.70 |
| **ERBB2 (V777L)** | 0.31 | | 0.43 | 0.21 | 54.40 |

A blank space means not detected.

Inhibitory Activity $IC_{50}$ Values (nM) of Series of Derivatives of Compound 1 (WO 2018/192536 A1) on EGFR Mutant Kinase, and the evaluation method was the same as above.

Compound A 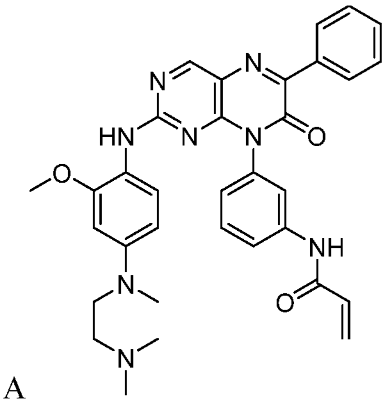 ; Compound B 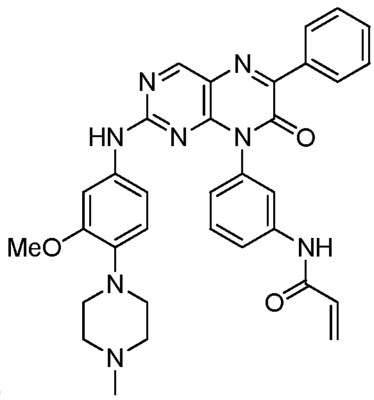 ;

Compound C 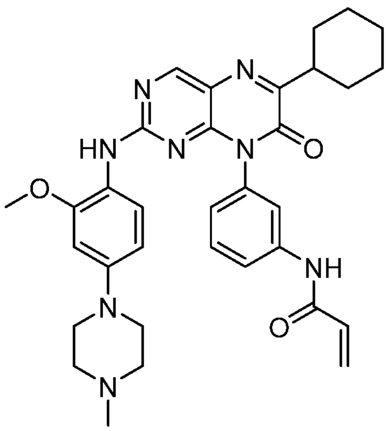 ; Compound D 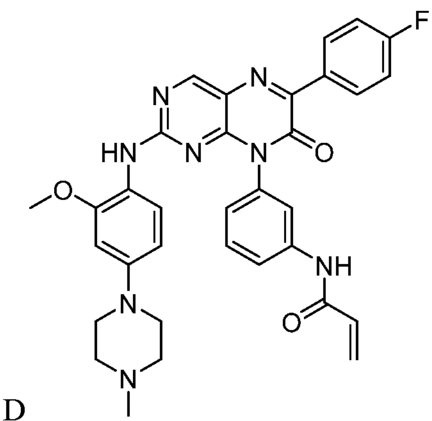 ;

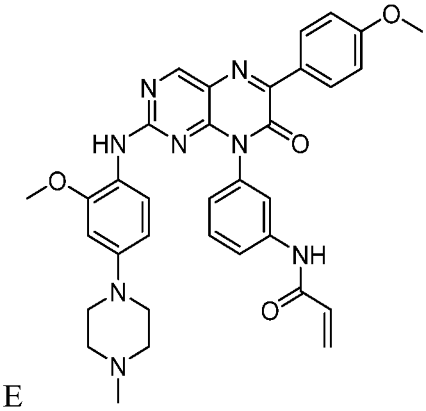

Compound E .

| Kinase | compound A | compound B | compound C | compound 1 | compound D | compound E |
|---|---|---|---|---|---|---|
| **EGFR (A763_Y764insFHEA)** | 0.67 | 1.56 | 0.94 | 0.40 | 0.57 | 0.77 |
| **EGFR (A763_Y764insFQEA)** | 0.35 | 0.78 | 0.45 | 0.29 | 1.92 | 2.37 |
| **EGFR (D761Y)** | 0.34 | 0.78 | 0.99 | 0.12 | 0.45 | 0.62 |
| **EGFR (D770_N771insNPG)** | 0.52 | 1.23 | 3.67 | 0.23 | 2.78 | 1.74 |
| **EGFR (D770_N771insNPG/T790M)** | 0.67 | 1.89 | 2.47 | 0.49 | 0.99 | 1.58 |
| **EGFR (D770GY)** | 0.28 | 0.76 | 0.97 | 0.14 | 0.87 | 1.01 |
| **EGFR (G719C)** | 1.09 | 1.04 | 1.79 | 0.51 | 2.41 | 1.64 |
| **EGFR (G719D)** | 2.19 | 0.82 | 3.78 | 0.28 | 2.91 | 1.64 |
| **EGFR (G719S)** | 1.45 | 0.97 | 2.75 | 0.36 | 3.02 | 1.67 |
| **EGFR (L861Q)** | 2.05 | 1,67 | 0.93 | 0.15 | 1.04 | 0.88 |
| **ERBB2 (D769Y)** | 0.79 | 0.77 | 4.58 | 0.35 | 0.78 | 2.59 |
| **ERBB2 (V777_G778insCG)** | 0.35 | 1.56 | 2.76 | 0.22 | 1.96 | 3.78 |
| **ERBB2 (V777L)** | 0.78 | 2.05 | 1.63 | 0.31 | 1.87 | 0.82 |

**Example 2. Detection Assay on Cell Line Proliferation Inhibition**

Mobocertinib (TAK-788, Takeda Pharmaceutical) has recently been granted Breakthrough Therapy Designation (BTD) drug status by U.S. FDA for treating patients with metastatic non-small cell lung cancer with insertion mutations of EGFR exon 20, whose disease has progressed after platinum-containing chemotherapy. For more objectively evaluating the activities of compound 1, TAK-788 was used as a positive control in this trial.
1. Proliferation inhibition assay of compound I on stable cell lines containing different EGFR mutations.
2. Project content
Inhibition of the proliferation of different mutant types of Ba/F3 EGFR cell lines by compound 1, TAK788, was examined by CTG Assay.
3. Main reagents and consumables
3. Compound: Compound 1 and TAK788, which were powders.
3. 2. List of Cells:

| Brand | Cat. No. | Name | Medium |
|---|---|---|---|
| Cobioer | CBP73176 | BA/F3_EGFR D769_N770insASV | RPMI-1640+10%FBS |
| Cobioer | CBP73175 | BA/F3_EGFR D770_N771insSVD | RPMI-1640+10%FBS |
| Cobioer | CBP73050 | BA/F3_EGFR L861Q | RPMI-1640+10%FBS |
| Cobioer | CBP73170 | BA/F3_EGFR G719A | RPMI-1640+10%FBS |
| Cobioer | CBP73180 | BA/F3_EGFR D770_N771insNPG | RPMI-1640+10%FBS |
| Cobioer | CBP73224 | BA/F3_EGFR H773_V774insH | RPMI-1640+10%FBS |

3.3 RPMI-1640 (Gibco #C11875500CP; Lot #8120069)
3.4 FBS (Gibco #10099-141C; Lot #2145068CP )
3.5 CellTiter-Glo® Luminescent Cell Viability Assay (Promega, Cat.No.: G7573; Lot #0000319784)
3.7 Cell Culture and Detection plate: 96 Well Asssay Plate (White Plate, Clear Bottom with Lid Tissue Covered Polystyrne 1/Pack, Costar 3610)
3.8 T25 Cell Culture flask (BioFiL # TCF 012050)
3.9 Compound formulation Plate (Costar # 3357)
4. Main experimental instruments
Synergy H1 multifunctional microplate reader (Biotek, REF:8040534; SN:500431)
5. Experimental principle
Ba/F3, a mouse pro-B cell line dependent on interleukin-3, is a common system for studying kinases and kinase inhibitors. Recombinant expression of some protein kinases can make Ba/F3 cells no longer dependent on IL-3 for growth, and based on such properties, the inhibition of a compound on the proliferation of Ba/F3 cells expressing different target kinases can be detected, so as to reflect inhibitory effects of the compound on different kinase targets at the cellular level for kinase inhibitor screening.
CellTiter-Glo® Luminescent Cell Viability Assay was employed in this experiment to detect cell proliferation, and the specific principles are as follows:
Adenosine Tri-Phosphate (ATP) is a common energy carrier used in various life activities in nature and is the smallest unit of energy storage and transfer. In the CellTiter-Glo™ Live Cell Assay Kit, fluorophore enzymes are used as detectors, which require the participation of ATP to generate light signals. After the CellTiter-Glo™ reagent is added to the cells, the luminescence value is measured. The light signal is proportional to the amount of ATP in the system, which is in turn positively correlated with the number of living cells. Therefore, by measuring the ATP content using the CellTiter-Glo kit, the viability of the cells and thus the level of cell proliferation can be reflected.

6. Experimental procedure

6.1 Cell Resuscitation and Culture

Cells were removed from the liquid nitrogen, quickly thawed in a water bath at 37 degree Celsius, lyophilization tubes were centrifuged at 1000 rpm, the supernatant of the lyophilized solution was discarded, and the cells were resuspended in RPMI1640+10% FBS medium and incubated in a cell culture incubator at 37 degree Celsius with a cell culture density maintaining at 2*10e5 to 2*10e6/ml at all times.

6.2 Formulation of Compound

1) The compound were serially diluted in DMSO in a compound formulation plate with a starting concentration of 1 or 10 mM and a gradient dilution of 3.
2) A new compound formulation plate was prepared, 198 ul of RPMI160+10% FBS medium was added to each well, 2 ul/well of compound from the previous gradient-diluted compound formulation plate was transferred to this plate and mixed well, so that the highest starting compound concentration in this plate was 1 or 10 uM, and diluted by gradient (this plate was a 10* compound concentration dilution plate).

6. 3. Detection Process

1) Cells in logarithmic growth phase were taken, the supernatant was discarded by centrifugation, and the cells were re-suspended in a fresh RPMI1640 medium at a cell density of 2*10e4/ml.
2) The re-suspended cells were inoculated into 2 96-well cell culture plates with white walls and clear bottom at 100 ul of cell suspension / well, and incubated overnight in a 37° C cell incubator.
3) The next day, one of the 96-well plates inoculated with cells were taken, 100ul/well of cell titer glo assay reagent was added for 60 minutes, the value was read and defined as G0 data.
4) Another parallel plate was taken, 11.1 ul/well of compound from the previously diluted 10* compound concentration dilution plate was added to this plate, so that the final concentration of compound in the cell assay plate started from 0.5 or 1 uM, which was 3-fold diluted for 9 concentration gradients. Additional DMSO control wells were set, and incubated in a 37° C cell incubator for another 72 hours.
5) The 96-well plate, in which cells were treated by the compound for 72 hrs, were taken from the incubator, 100 ul/well of cell titer glo assay reagent was added for 60 minutes, the value was read and defined as G3 data.
6) The cell proliferation rate corresponding to each well was calculated according to the following formula

% prolifation = (G3 of the well of the compound to be tested - mean value of G0) / (mean value of G3 of the DMSO control well − mean value of G0) * 100.

7) Based on the proliferation rate and concentration thereof corresponding to each gradient concentration well, a gradient curve of cell proliferation was fitted using Prism Graphpad 5.0 software and the GI50 of the compound was calculated (GI50 is defined as the concentration of the compound corresponding to a cell proliferation rate of 50%) and the fitting formula in the software is as follows:

Y=Bottom + (Top-Bottom)/(1+10^((LogIC50-X)*HillSlope))

7. Experimental Results:

GI50 values of compound I in the proliferation inhibition assay against stable cell lines containing different EGFR mutations are shown in the table below:

| Strain | GI50 ( nM) | |
|---|---|---|
| | Compound 1 | TAK788 |
| **EGFR V769_D770insASV BaF3** | 1.63 | 1.49 |
| **EGFR D770_N771insNPG BaF3** | 0.99 | 1.53 |
| **EGFR D770_N771insSVD BaF3** | 5.59 | 4.73 |
| **EGFR H773_V774insH BaF3** | 5.42 | 4.44 |
| **EGFR G719A BaF3** | 0.58 | 4.66 |

(continued)

| Strain | GI50 ( nM) | |
|---|---|---|
| | Compound 1 | TAK788 |
| **EGFR L861Q BaF3** | 0.98 | 2.55 |
| | | |

Figures 1a-f show GI50 values of compound I in the proliferation inhibition assay against stable cell lines containing different EGFR mutations, wherein compound 1 is shown as R201 and compound TAK788 is shown as R-203.
CONCLUSION: The results of compound 1 and a series of derivatives thereof against EGFR non-classical mutant kinase activity indicate that compound 1 possesses excellent inhibitory activities; and further proliferation-inhibiting activities against classical EGFR 20ins mutant stable cell lines indicates that compound 1 reaches or is better than the EGFR 20ins kinase inhibitor TAK788, which is now generally considered to be the most promising.
All literature referred to in the present invention is cited by reference in the present application as if each literature was cited separately by reference. It should furthermore be understood that after reading the above teachings of the invention, a skilled person may make various alterations or modifications to the invention, and such equivalent forms also fall within the scope defined by the claims appended to this application.

## Claims

1. Use of a compound of formula I or a pharmaceutically acceptable salt thereof in the preparation of a drug for inhibiting mutant EGFR or a drug for treating or preventing mutant EGFR-mediated diseases:

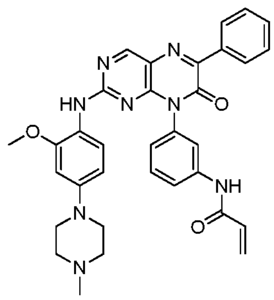

I.

2. The use of claim 1, wherein the mutant EGFR comprises at least one of the following mutations: point mutation and insertion mutation of EGFR 18-21 exon, and point mutation and insertion mutation of ErbB 2.

3. The use of claim 2, wherein the mutant EGFR comprises:

   18 exon G719X, E709X, K716A, K728A point mutations and deletion mutation at codon 709
   19 exon insertion mutations I744-K745InsKIPVAI, K745-E746insIPVAIK, K745-E746insVPVAIK, K745-E746insTPVAIK and point mutation D761Y;
   20 exon insertion mutation and point mutation include: A763-Y764insFQEA, A763-Y764insFHEA, V769-D770insASV, V769-D770insDNP , D770-N771insNPG, D770-N771insNPH, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV and the like, and 20 exon point mutation S768I;
   21 exon point mutation L861Q;
   ERBB2 point mutations V777L, D769Y, R896C, P 1170A and insertion mutations V777-G778insCG, P780-Y781insGSP, and the like.

4. The use of claim 2, wherein the mutant EGFR comprises at least one of the following mutations: G719X (X represents A, S, C, D), D761Y, A763-Y764insFQEA, A763-Y764insFHEA, V769-D770insASV, D770-N771insSVD, D770-N771insASVDN, D770-N771insG, N771-P772insSVDNP, N771-H773dupNPH, P772-H773insPNP, P772-H773insPR, H773-V774insH, A763-Y764insFQEA, H773-V774insPH, H773-V774insNPH, N771-P772insH, H771-P772insN, H773-V774insAH, D770delinsGY, V774-C775insHV, L861Q, V777-G778insCG, V777L, D769Y and the like.

**5.** The use of claim 4, wherein the mutant EGFR comprises at least one of the following mutations: A763_Y764insFHEA, A763-Y764insFQEA, d747-749/A750P D761Y, D770-N771insNPG, D770-N771insNPG/T790M, D770GY, G719C, G719D, G719S, and L861Q, and V777-G778insCG, V777L, D769Y of ERBB, and the like.

**6.** The use of claim 1, wherein the disease is a cancer.

**7.** The use of claim 6, wherein the cancer is non-small cell lung cancer, small cell lung cancer, lung adenocarcinoma, lung squamous cell carcinoma, breast cancer, pancreatic cancer, prostate cancer, ovarian cancer, glioblastoma, head and neck squamous cell carcinoma, cervical cancer, esophageal cancer, liver cancer, kidney cancer, colon cancer, skin cancer, leukemia, lymphoma, gastric cancer or multiple myeloma.

**8.** A compound of formula I or a pharmaceutically acceptable salt thereof, for inhibiting mutant EGFR or treating or preventing mutant EGFR-mediated diseases,

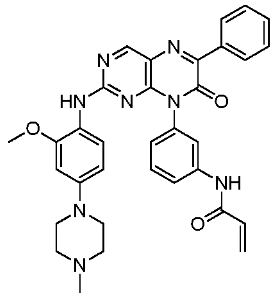

I.

**9.** A method for inhibiting mutant EGFR or treating or preventing mutant EGFR-mediated diseases, comprising a step of administering a compound of formula I or a pharmaceutically acceptable salt thereof to a subject in need thereof,

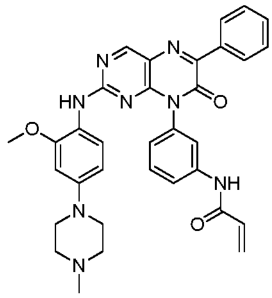

I.

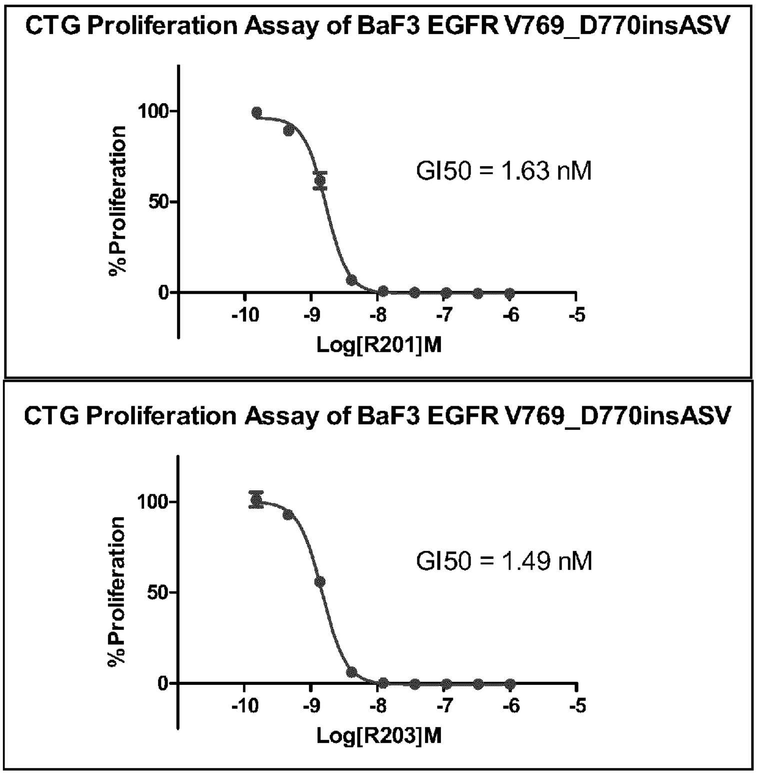
CTG Proliferation Assay of BaF3 EGFR V769_D770insASV
% Proliferation
GI50 = 1.63 nM
Log[R201]M
CTG Proliferation Assay of BaF3 EGFR V769_D770insASV
% Proliferation
GI50 = 1.49 nM
Log[R203]M

Fig. 1a

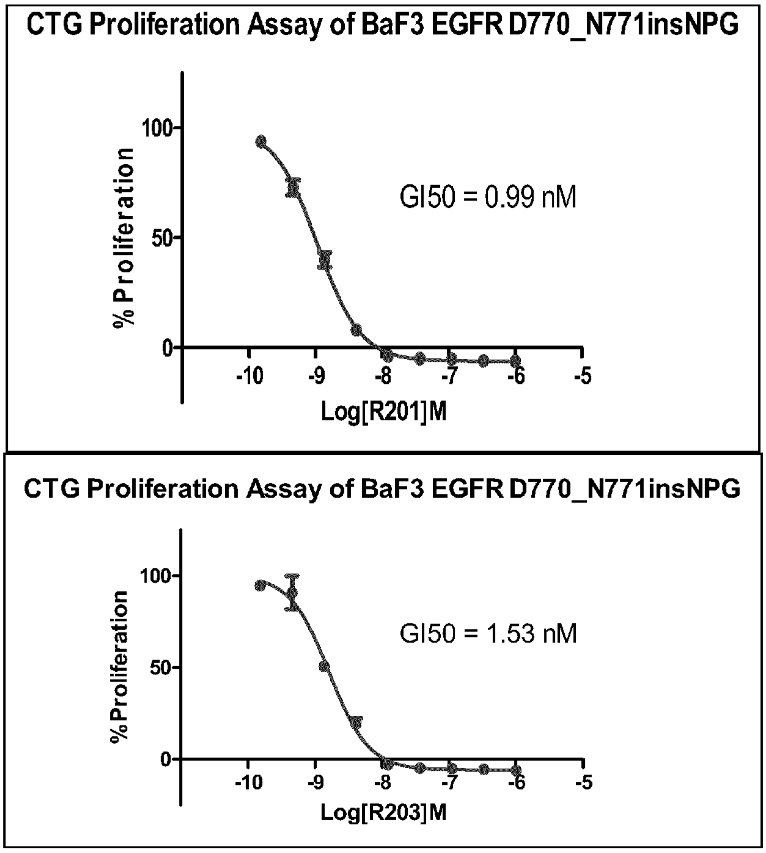
CTG Proliferation Assay of BaF3 EGFR D770_N771insNPG
% Proliferation
GI50 = 0.99 nM
Log[R201]M
CTG Proliferation Assay of BaF3 EGFR D770_N771insNPG
% Proliferation
GI50 = 1.53 nM
Log[R203]M

Fig. 1b

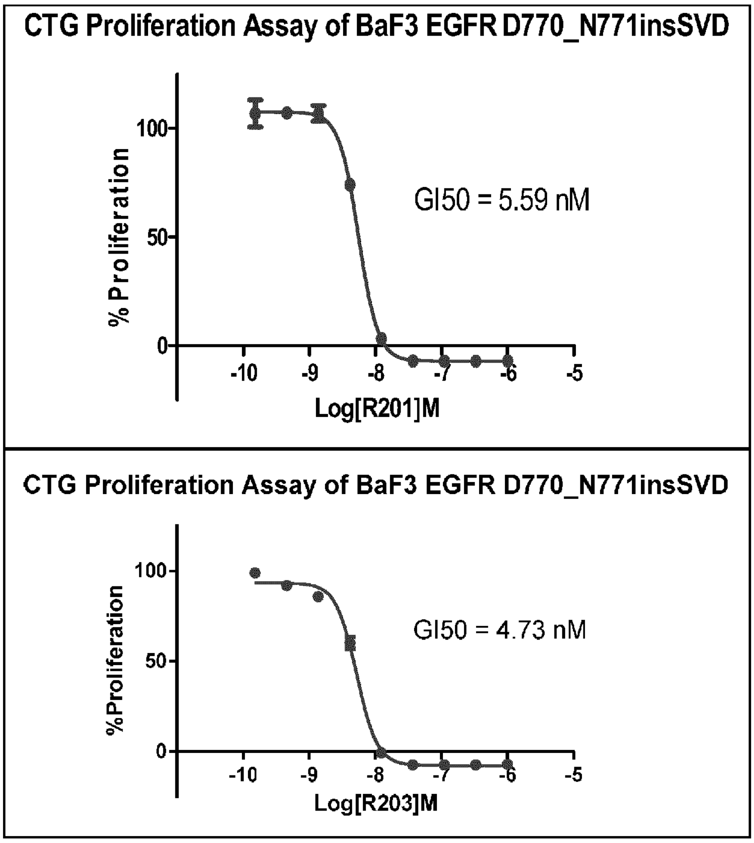
CTG Proliferation Assay of BaF3 EGFR D770_N771insSVD
% Proliferation
100
50
0
-10
-9
-8
-7
-6
-5
Log[R201]M
GI50 = 5.59 nM
CTG Proliferation Assay of BaF3 EGFR D770_N771insSVD
%Proliferation
100
50
0
-10
-9
-8
-7
-6
-5
Log[R203]M
GI50 = 4.73 nM

Fig. 1c

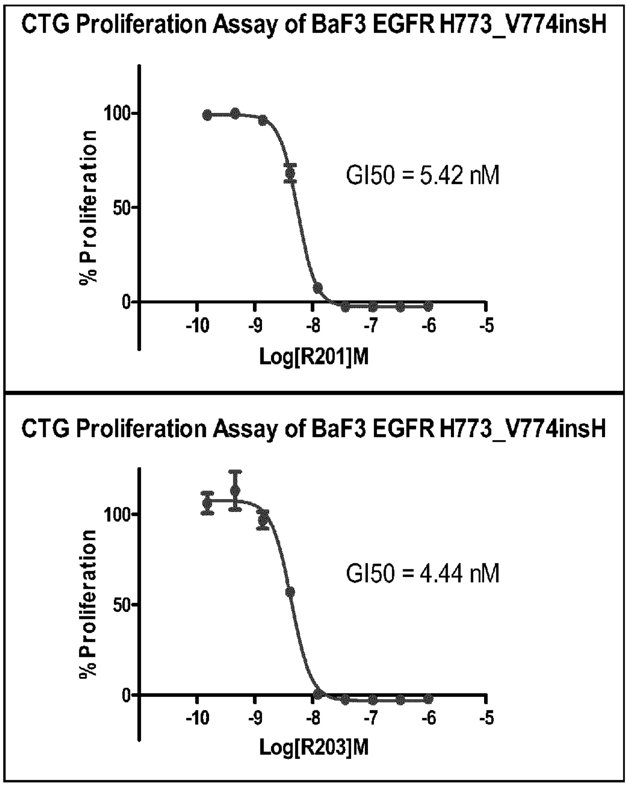
CTG Proliferation Assay of BaF3 EGFR H773_V774insH
% Proliferation
100
50
0
-10
-9
-8
-7
-6
-5
Log[R201]M
GI50 = 5.42 nM
CTG Proliferation Assay of BaF3 EGFR H773_V774insH
% Proliferation
100
50
0
-10
-9
-8
-7
-6
-5
Log[R203]M
GI50 = 4.44 nM

Fig. 1d

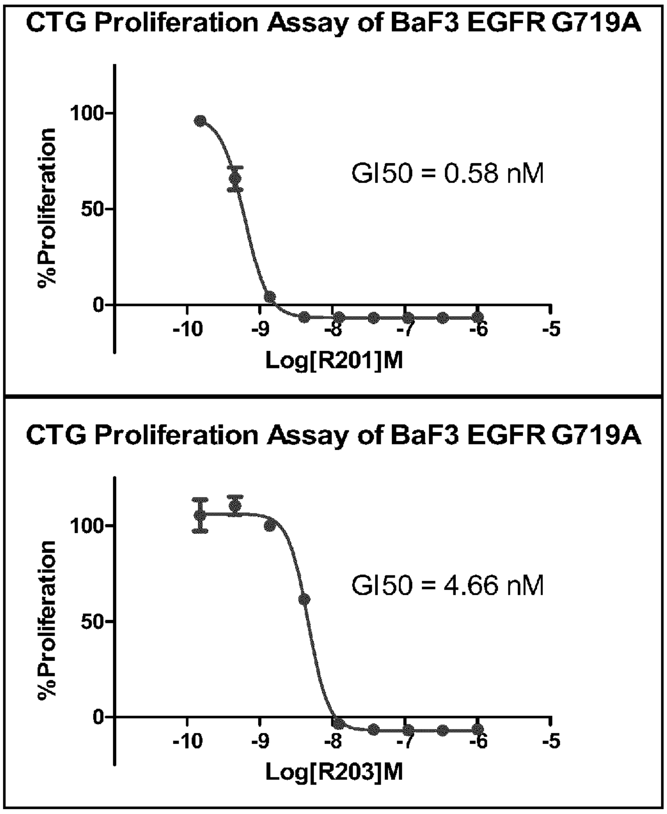
CTG Proliferation Assay of BaF3 EGFR G719A
100
50
0
%Proliferation
-10
-9
-8
-7
-6
-5
Log[R201]M
GI50 = 0.58 nM
CTG Proliferation Assay of BaF3 EGFR G719A
100
50
0
%Proliferation
-10
-9
-8
-7
-6
-5
Log[R203]M
GI50 = 4.66 nM

Fig. 1e

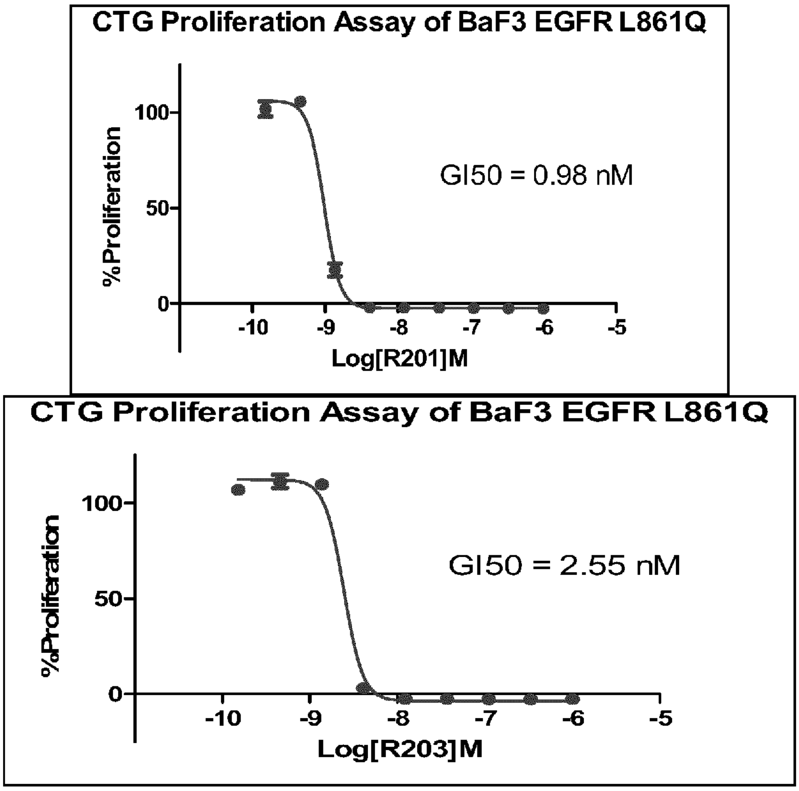
CTG Proliferation Assay of BaF3 EGFR L861Q
100
50
0
%Proliferation
-10
-9
-8
-7
-6
-5
Log[R201]M
GI50 = 0.98 nM
CTG Proliferation Assay of BaF3 EGFR L861Q
100
50
0
%Proliferation
-10
-9
-8
-7
-6
-5
Log[R203]M
GI50 = 2.55 nM

Fig. 1f

**INTERNATIONAL SEARCH REPORT**

International application No.
**PCT/CN2021/116076**

**A. CLASSIFICATION OF SUBJECT MATTER**

A61K 31/519(2006.01)i; A61K 31/5365(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , NCBI, baidu, STN, 华东理工大学, 李洪林, 蝶啶酮, pteridinone, EGFR, 突变, mutation, 2248215-32-7/rn

**C. DOCUMENTS CONSIDERED TO BE RELEVANT**

| Category* | Citation of document, with indication, where appropriate, of the relevant passages | Relevant to claim No. |
|---|---|---|
| X | WO 2016192609 A1 (EAST CHINA UNIVERSITY OF SCIENCE AND TECHNOLOGY) 08 December 2016 (2016-12-08)<br>description abstract, claims 1, 3, 5-10, page 52 table 1 | 1-9 |
| A | WO 2013170671 A1 (EAST CHINA UNIVERSITY OF SCIENCE AND TECHNOLOGY) 21 November 2013 (2013-11-21)<br>claims 1-10 | 1-9 |
| A | WO 2018192536 A1 (EAST CHINA UNIVERSITY OF SCIENCE AND TECHNOLOGY et al.) 25 October 2018 (2018-10-25)<br>claims 1-14 | 1-9 |
| A | WO 2015188738 A1 (HEFEI INSTITUTES OF PHYSICAL SCIENCE, CHINESE ACADEMY OF SCIENCES et al.) 17 December 2015 (2015-12-17)<br>claims 1-14 | 1-9 |
| A | WO 2015003658 A1 (BETTA PHARMACEUTICALS CO., LTD.) 15 January 2015 (2015-01-15)<br>claims 1-67 | 1-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 November 2021** | **25 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/116076**

| C. DOCUMENTS CONSIDERED TO BE RELEVANT | | |
|---|---|---|
| Category* | Citation of document, with indication, where appropriate, of the relevant passages | Relevant to claim No. |
| A | CN 109305967 A (SHANGHAI INSTITUTE OF MATERIA MEDICA, CHINESE ACADEMY OF SCIENCES) 05 February 2019 (2019-02-05)<br>claims 1-10 | 1-9 |

Form PCT/ISA/210 (second sheet) (January 2015)

**INTERNATIONAL SEARCH REPORT**

International application No.
**PCT/CN2021/116076**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9**
because they relate to subject matter not required to be searched by this Authority, namely:

[1] Claim 9 relates to a method for inhibiting a mutant EGFR or treating or preventing mutant EGFR-mediated diseases, and thus belongs to treatment methods for diseases under PCT Rule 39.1(iv). However, a search has still been carried out on the basis of a pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/116076**

| Patent document cited in search report | | | Publication date (day/month/year) | Patent family member(s) | | | Publication date (day/month/year) |
|---|---|---|---|---|---|---|---|
| WO | 2016192609 | A1 | 08 December 2016 | EP | 3312180 | A1 | 25 April 2018 |
| | | | | CN | 107922417 | A | 17 April 2018 |
| | | | | EP | 3312180 | A4 | 26 December 2018 |
| | | | | CA | 2987466 | A1 | 08 December 2016 |
| | | | | AU | 2016272057 | A1 | 25 January 2018 |
| | | | | KR | 20180021743 | A | 05 March 2018 |
| | | | | JP | 2018516278 | A | 21 June 2018 |
| | | | | AU | 2016272057 | B2 | 08 October 2020 |
| | | | | CN | 106279173 | A | 04 January 2017 |
| | | | | US | 2018148454 | A1 | 31 May 2018 |
| WO | 2013170671 | A1 | 21 November 2013 | DE | 112013002484 | T5 | 29 January 2015 |
| | | | | CN | 106008511 | A | 12 October 2016 |
| | | | | CN | 106008511 | B | 14 August 2018 |
| | | | | US | 9670213 | B2 | 06 June 2017 |
| | | | | CN | 103930425 | B | 27 April 2016 |
| | | | | US | 2015126508 | A1 | 07 May 2015 |
| | | | | CN | 103930425 | A | 16 July 2014 |
| | | | | JP | 2015516445 | A | 11 June 2015 |
| | | | | JP | 6114820 | B2 | 12 April 2017 |
| WO | 2018192536 | A1 | 25 October 2018 | CN | 108721298 | A | 02 November 2018 |
| WO | 2015188738 | A1 | 17 December 2015 | CN | 104825455 | B | 15 August 2017 |
| | | | | CN | 104825455 | A | 12 August 2015 |
| WO | 2015003658 | A1 | 15 January 2015 | EP | 3019489 | A4 | 28 December 2016 |
| | | | | HK | 1221953 | A1 | 16 June 2017 |
| | | | | JP | 6510510 | B2 | 08 May 2019 |
| | | | | US | 2018009782 | A1 | 11 January 2018 |
| | | | | JP | 2016525509 | A | 25 August 2016 |
| | | | | AU | 2014289762 | B2 | 16 February 2017 |
| | | | | TW | 201512176 | A | 01 April 2015 |
| | | | | CA | 2917735 | A1 | 15 January 2015 |
| | | | | US | 10059688 | B2 | 28 August 2018 |
| | | | | US | 2016145237 | A1 | 26 May 2016 |
| | | | | RU | 2656591 | C2 | 06 June 2018 |
| | | | | US | 9783524 | B2 | 10 October 2017 |
| | | | | EP | 3019489 | A1 | 18 May 2016 |
| | | | | AU | 2014289762 | A1 | 18 February 2016 |
| | | | | TW | I610923 | B | 11 January 2018 |
| | | | | SG | 11201600055 P | A | 26 February 2016 |
| | | | | RU | 2016103938 | A | 16 August 2017 |
| | | | | CA | 2917735 | C | 18 April 2017 |
| CN | 109305967 | A | 05 February 2019 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108721298 A
- WO 2018192536 A1


### Non-patent literature cited in the description


- *Cancer Sci.,* 2016, vol. 107 (9), 1179-1186